(19) Europäisches Patentamt
European Patent Office
Office européen des brevets

(11) **EP 1 712 126 B1**

(12) **EUROPEAN PATENT SPECIFICATION**

(45) Date of publication and mention
of the grant of the patent:
**11.01.2012 Bulletin 2012/02**

(21) Application number: **04820979.5**

(22) Date of filing: **05.11.2004**

(51) Int Cl.:
*A01K 67/027* [(2006.01)]   *C12N 15/09* [(2006.01)]
*C12P 21/02* [(2006.01)]

(86) International application number:
**PCT/JP2004/016438**

(87) International publication number:
**WO 2005/065450 (21.07.2005 Gazette 2005/29)**

(54) **METHOD OF CONSTRUCTING A TRANSGENIC BIRD.**

VERFAHREN ZUR HERSTELLUNG EINES TRANSGENEN VOGELS.

PROCÉDÉ POUR LA PRODUCTION D'UN OISEAU TRANSGÉNIQUE.

(84) Designated Contracting States:
**AT BE BG CH CY CZ DE DK EE ES FI FR GB GR HU IE IS IT LI LU MC NL PL PT RO SE SI SK TR**

(30) Priority: **08.01.2004 JP 2004003045**

(43) Date of publication of application:
**18.10.2006 Bulletin 2006/42**

(73) Proprietors:
• **KANEKA CORPORATION**
**Osaka-shi, Osaka 530-8288 (JP)**
• **Nagoya University**
**Nagoya-shi, Aichi 464-8601 (JP)**

(72) Inventors:
• **YAMASHITA, Takashi**
**Akashi-shi,**
**Hyogo 674-0084 (JP)**
• **SHINDO, Takuya**
**Kobe-shi,**
**Hyogo 654-0055 (JP)**
• **IIJIMA, Shinji**
**Nagoya-shi,**
**Aichi 468-0022 (JP)**
• **KAMIHIRA, Masamichi**
**Chikusa-ku,**
**Nagoya-shi,**
**Aichi 464-0846 (JP)**
• **NISHIJIMA, Kenichi**
**Chikusa-ku,**
**Nagoya-shi,**
**Aichi 464-0804 (JP)**

(74) Representative: **Gillet, Raphaëlle et al**
**Cabinet Beau de Loménie**
**158, rue de l'Université**
**75340 Paris Cedex 07 (FR)**

(56) References cited:
EP-A- 1 672 076    WO-A-99/19472
WO-A-2004/016081    JP-A- 2002 176 880

• HARVEY A J ET AL: "Expression of exogenous protein in the egg white of transgenic chickens" NATURE BIOTECHNOLOGY, NATURE PUBLISHING GROUP, NEW YORK, NY, US, vol. 19, 1 April 2002 (2002-04-01), pages 396-399, XP002973268 ISSN: 1087-0156
• MIZUARAI S ET AL: "Production of transgenic quails with high frequency of germ-line transmission using VSV-G pseudotyped retroviral vector" BIOCHEMICAL AND BIOPHYSICAL RESEARCH COMMUNICATIONS, ACADEMIC PRESS INC. ORLANDO, FL, US, vol. 286, no. 3, 1 August 2001 (2001-08-01), pages 456-463, XP002973599 ISSN: 0006-291X
• PETITTE JAMES N: "The avian germline and strategies for the production of transgenic chickens." JOURNAL OF POULTRY SCIENCE, vol. 39, no. 4, October 2002 (2002-10), pages 205-228, XP002533188 ISSN: 1346-7395
• HAMBURGER V ET AL: "A series of normal stages in the development of the chick embryo" JOURNAL OF MORPHOLOGY (1931. PRINT) 1951, vol. 88, no. 1, 1951, pages 49-92, XP002533189 ISSN: 0362-2525

- KAMIHIRA MASAMICHI ET AL: "High-level expression of single-chain Fv-Fc fusion protein in serum and egg white of genetically manipulated chickens by using a retroviral vector" JOURNAL OF VIROLOGY, THE AMERICAN SOCIETY FOR MICROBIOLOGY, US, vol. 79, no. 17, 1 September 2005 (2005-09-01), pages 10864-10874, XP002474041 ISSN: 0022-538X
- ONO K. ET AL: 'scFv-Fc Yugo Tanpakushitsu o Seisan suru Transgenic Uzura no Sakusei.' THE SOCIETY OF CHEMICAL ENGINEERS, SHUKI TAIKAI KENKYU HAPPYO KOEN YOSHISHU vol. 36, 2003, page 780, XP002990217
- KAMIHIRA M. ET AL: 'Transgenic Niwatori ni yoru Ko-hito CD2 Chimera Kotai no Seisan.' THE SOCIETY OF CHEMICAL ENGINEERS, SHUKI TAIKAI KENKYU HAPPYO KOEN YOSHISHU vol. 36, 2003, page 781, XP002990218
- KAMIHIRA M. ET AL: 'Trangenic Niwatori ni yoru Monoclonal Kotai no Seisan.' THE MOLECULAR BIOLOGY SOCIETY OF JAPANN NENKAI PROGRAM KOEN YOSHISHU vol. 26, 2003, page 791, XP002990219
- MATSUI ET AL.: "Proviral silencing in embryonic stem cells requires the histone methyltransferase ESET", NATURE, vol. 464, 2010, pages 927-931,

Remarks:
  The file contains technical information submitted after the application was filed and not included in this specification

SEQUENCE LISTING

<110> Kaneka Corporation
<120> TRANSGENIC BIRD AND METHOD OF CONSTRUCTING THE SAME

<130> B030497W001

<150> JP 2004-003045
<151> 2004-1-8

<160> 18

<210> 1
<211> 28
<212> DNA
<213> Artificial Sequence

<220>
<223> Des i gned sequence of a 5'-primer incorporating the SalI recognition site at the 5' terminal used for PCR amplification of the chicken b-actin promoter fragment lacking the intron

<400> 1
acgcgtcgac gtgcatgcac gctcattg          28

<210> 2
<211> 26
<212> DNA
<213> Artificial Sequence

<220>
<223> Des i gned sequence of a 3'-primer i ncorporat i ng the Sall recognition site at the 5' terminal used for PCR amplification of the chicken b-actin promoter fragment lacking the intron

<400> 2
acgcgtcgac aacgcagcga ctcccg 26

<210> 3
<211> 61
<212> DNA
<213> Artificial Sequence

<220>
<223> Designed oligonucleotide acting as a sense chain in annealing to construct the coding fragment of the chicken lysozyme secretion signal

<400> 3

ctagaccatg aggtctttgc taatcttggt gctttgcttc ctgcccctgg ctgctctggg 60

g 61

<210> 4
<211> 57
<212> DNA
<213> Artificial Sequence

<220>
<223> Designed oligonucleotide acting as an anti-sense chain in annealing to construct the coding fragment of the chicken lysozyme secretion signal

<400> 4
ccccagagca gccaggggca ggaagcaaag caccaagatt agcaaagacc tcatggt 57

<210> 5
<211> 26
<212> DNA
<213> Artificial Sequence

<220>
<223> Des i gned sequence of a 5'-primer i ncorporat i ng the Dra I recognition site at the 5' terminal used for PCR amplification of the scFv coding fragment

<400> 5
gcgtttaaag tgacgttgga cgtccg          26

<210> 6

<211> 29
<212> DNA
<213> Artificial Sequence

<220>
<223> Designed sequence of a 3' -primer i ncorporat i ng the BamH I recognition site at the 5' terminal used for PCR amplification of the scFv coding fragment

<400> 6
attaggatcc gcgcttaagg acggtcagg          29

<210> 7
<211> 18
<212> DNA
<213> Artificial Sequence

<220>
<223> Designed sequence of a 5'-primer used for PCR amplification of the coding fragment of the human antibody heavy chain γ1 constant region

<400> 7
caagcttcaa gggcccat          18

<210> 8
<211> 19
<212> DNA
<213> Artificial Sequence

<220>
<223> Designed sequence of a 3'-primer used for PCR amplification of the coding fragment of the human antibody heavy chain γ1 constant region

<400> 8
atttacccgg agacaggga          19

<210> 9
<211> 35
<212> DNA
<213> Artificial Sequence

<220>
<223> Designed sequence of a 5'-primer incorporating the BamH I recognition site at the 5' terminal used for PCR amplification of the coding fragment of the human antibody heavy chain γ1 Fc region

<400> 9
attaggatcc gagcccaaat cttgtgacaa aactc          35

<210> 10
<211> 30
<212> DNA
<213> Artificial Sequence

<220>
<223> Designed sequence of a 3'-primer incorporating the Hind III recognition site at the 5' terminal used for PCR amplification of the coding fragment of the human antibody heavy chain γ1 Fc region

<400> 10
agcaagcttt catttacccg gagacaggga          30

<210> 11
<211> 30
<212> DNA
<213> Artificial Sequence

<220>
<223> Des i gned sequence of a 5'-primer used for PCR amplification of a 393 bp fragment in the gene of scFv

<400> 11
gtcttattag cggtgctggt agtagcacaa          30

<210> 12
<211> 25
<212> DNA
<213> Artificial Sequence

<220>
<223> Des i gned sequence of a 3'-primer used for PCR amplification of a 393 bp fragment in the gene of scFv

<400> 12
gagacttctg ctggtaccag ccata          25

<210> 13
<211> 30
<212> DNA
<213> Artificial Sequence

<220>
<223> Des i gned sequence of a 5'-primer used for PCR amplification of a 311 bp fragment in the gene of GFP

<400> 13
agctcaccct gaaattcatc tgcaccactg          30

<210> 14
<211> 30
<212> DNA
<213> Artificial Sequence

<220>
<223> Designed sequence of a 3'-primer used for PCR amplification of a 311 bp fragment in the gene of GFP

<400> 14
gttgtattcc agcttgtggc cgagaatgtt          30

<210> 15
<211> 27
<212> DNA
<213> Artificial Sequence

<220>
<223> Des i gned sequence of a 5'-primer used for PCR amplification of a 355 bp fragment in the gene of GFP

<400> 15
caacactggt cactaccttc acctatg          27

<210> 16
<211> 25
<212> DNA

<213> Artificial Sequence

<220>
<223> Des i gned sequence of a 3'-primer used for PCR amplification of a 355 bp fragment in the gene of GFP

<400> 16
acggatccat cctcaatgtt gtgtc          25

<210> 17
<211> 26
<212> DNA
<213> Artificial Sequence

<220>
<223> Des i gned sequence of a 5'-primer used for PCR amplification of a 317 bp fragment in the gene of ovalbumin

<400> 17
cgctttgata aacttccagg attcgg          26

<210> 18
<211> 27
<212> DNA
<213> Artificial Sequence

<220>
<223> Des i gned sequence of a 3'-primer used for PCR amplification of a 317 bp fragment in the gene of ovalbumin

<400>18
catctagctg tcttgcttaa gcgtaca          27

## Description

TECHNICAL FIELD

The present invention relates to a method for constructing a transgenic bird which is applicable in the production of a physiologically active protein.

BACKGROUND ART

As a result of the progresses in gene recombination technology, a large number of physiologically active proteins have been produced commercially for use as drugs and in other uses. However, such microorganisms as *Escherichia coli* that have been used most frequently in producing such recombinant proteins are incapable of adding a sugar chain thereto and, therefore, the proteins produced cannot undergo modification by sugar chain addition; hence, disadvantageously, they show decreases in activity as compared with their intrinsic activity and/or show deteriorations in stability.

Furthermore, microorganisms cannot produce complicated proteins composed of a plurality of units, for example antibodies. It is a great drawback that they cannot be utilized for producing monoclonal antibodies to be used as drugs.

Therefore, those physiologically active proteins such as erythropoietin and antibodies used as drugs, which are required to have a sugar chain, are currently produced by means of cultured cell reactors. However, such production processes using animal cells entail higher costs as compared with the use of microorganisms and, therefore, it is a problem that a high load is imposed on patients and on the administration.

A novel method for producing proteins while overcoming these drawbacks that has attracted attention comprises utilizing transgenic animals. The proteins produced in milk, blood or eggs by transgenic animals can have a sugar chain or chains added and, in addition, the production cost is one tenth to one hundredth as compared with the case of using cultured animal cells (Nature Biotechnology 19, 184, 2001). The transgenics using birds, for example, are expected to be put to practical use because of such advantageous features as the shortness in period until maturity and the smallness in breeding area.

While the technology of protein production utilizing transgenic birds has such and other advantages, some difficulties are encountered in putting that technology to practical use. One of them is the difficulty in introducing a foreign gene into fertilized avian eggs (Harvey A. J. et al. Consistent production of transgenic chickens using replication-deficient retroviral vectors and high-throughput screening procedures. Poult. Sci., Feb. 2002, Vol. 81, No. 2, pp. 202-212). The present inventors previously succeeded in efficiently constructing transgenic chimeras (referred to as "G0") carrying a transgene partially in their bodies as a result of microinjection of the gene into avian early embryos using a highly safe, replication-deficient viral vector (Japanese Kokai Publication 2002-176880).

However, the gene introduced in this manner undergoes inactivation by the biophylactic mechanisms of the host (phenomenon called silencing), failing to produce the desired protein or producing the protein, if it is expressed, only in trace amounts. Furthermore, for supplying the protein stably as a medicine, it is necessary to establish an avian line having a transgene in all somatic cells of the body as offspring of the G0 transgenic chimera. These offspring of the G0 transgenic are called G1, G2, G3 and the like in order of generation.

SUMMARY OF THE INVENTION

In view of the above-mentioned state of the art, it is an object of the present invention to establish an efficient method for constructing transgenic birds having a transgene in all somatic cells and capable of expressing the protein encoded by the transgene at high levels for the purpose of applying the transgenic birds as a safe and efficient protein production system.

The invention is a method as specified in claim 1.

DETAILED DESCRIPTION OF THE INVENTION

The invention is a method as specified in claim 1. When injected with the vector into the early heart formed in the embryo after the lapse of a certain period of time specifiable depending on the avian species, the injection target early embryo can give a transgenic bird capable of effectively expressing the gene in question.

The gene to be introduced into a bird to give a transgenic bird is an antibody gene.

When a chicken or quail is used as the bird into which the gene is to be introduced, the desired protein can be obtained in the eggs in a productive and stable manner. Further embodiments of the invention are defined in the dependent claims.

Although the locus, on chromosome, of the gene introduced by microinjection cannot be specified, it is possible to identify G0 parents capable of efficiently producing G1 birds by sorting out reproductive lineage chimeras that have the gene inserted in the reproductive system from among the G0 transgenic chimeras constructed. Effective in such sorting out is a method comprising collecting sperms from G0 males by abdominal massaging and testing the sperms for the occurrence of the transgene therein. This sorting method also constitutes an aspect of the present disclosure.

The invention thus discloses a method for constructing transgenic birds of advantage in antibody production.

The transgenic bird constructed by that method can be utilized in producing antibody that can be utilized as a drug, and the like.

In the following, the invention is described in detail.

The G1 transgenic bird and/or an offspring thereof is a bird with a foreign gene introduced therein by means of a replication-deficient retroviral vector and is characterized in that it contains the foreign gene evenly in somatic cells thereof and expresses the desired protein originating in the transgene stably in the blood thereof or in the egg white or yolk.

The bird to be used in the practice of the invention is not particularly restricted but there may be mentioned, for example, chickens, turkeys, ducks, ostriches, quails and other domestic birds and pet birds domesticated for the purpose of eating meat and/or ovum collection. Among them, chickens and quails are readily available and fecund as egg laying species, hence are preferred.

As the retroviral vector to be used in the practice of the invention, there may be mentioned vectors derived from Moloney murine leukemia virus (MoMLV), avian leukosis virus (ALV) and the like, and lentivirus vectors, and the like. Among them, MoMLV-derived ones are preferred, although the retroviral vector to be used is not limited thereto.

From the safety viewpoint, a vector deficient in ability for autoreplication as a result of the deficiency of any or all of the three genes gag, pol and env, which are necessary for the replication of viral particles, is generally used as the vector for gene transfer. Preferred for efficiently infecting bird cells with this viral vector is a viral vector with the coat protein thereof artificially VSV-G (vesicular stomatitis virus-derived) pseudotyped. The viral vector to be used in the practice of the invention is not limited to this viral type, however.

The pseudotype viral vector prepared by utilizing packaging cells, a helper virus or the like is introduced into the early embryo, blood vessel and heart by the general microinjection technique (Bosselman, R. A. et al. (1989), Science, 243, 533). Conceivable as other gene transfer techniques are the lipofection and electroporation techniques, and the like.

The gene to be introduced into birds in the practice of the invention may be constituted of a marker gene, the structural gene for the expression of an antibody a promoter gene for controlling the expression of such genes and a secretion signal gene.

The marker gene is, for example, the neomycin resistance gene, the β-galactosidase gene, or the gene coding for a fluorescent protein such as GFP (green fluorescent protein).

The structural gene for the expression of a desired antibody is useful in the genetic industry, for example encodes human monoclonal antibodies.

In particular, structural genes for extrinsic antibodies, for example genes for antibodies having the human IgG class constant region or genes for antibodies having the human IgG1, quail IgG2, chicken IgG2 or mouse IgG2 subclass constant region are preferred in view of good accumulability of such antibodies in eggs.

As the above-mentioned structural genes that are preferred, there may be mentioned structural genes for chimeric antibodies. The term "chimeric antibody" refers to an antibody having two or more different inherited characters. The antibodies for medical use as produced by murine hybridomas have a problem in that when administered into the human body, they cause the rejection reaction by the immune system since they are of the murine origin. For overcoming this disadvantage, the Fc portion etc. of a murine antibody is humanized, for example, by the recombination technique, whereby the risk of rejection can be markedly reduced.

As the chimeric antibodies mentioned above, there may be mentioned, for example, anti-human CD2 antibodies, anti-CD 20 receptor antibodies, and anti-TNF antibodies. Some of them have already been marketed as medicines.

A more preferred example of the above-mentioned structural gene is the structural gene for scFv-Fc antibody. As recombinant antibodies for medical use, there are also a group of the so-called low-molecular antibodies. Immunoglobulin IgG comprises a domain consisting of a VH-VL hetero dimer as called variable region (Fv: fragment of variable region) directly binding to an antigen, and this Fv domain has by itself a sufficient level of antigen-binding ability in spite of its being about one fifth in molecular weight as compared with IgG. The product of artificial binding together of the VH and VL domains by means of a peptide linker is a low-molecular antibody called single-chain antibody (scFv: single chain Fv), which has been known to be improved in stability as compared with the VH or VL alone.

Powers et al. (Powers, D. B. et al. (2000), J. Immunol. Methods, 251, 123) found that when this scFv is fused to the human IgG1-derived Fc portion, the stability in blood is enhanced. This scFv-Fc antibody is thought to be useful for medical purposes but cannot be produced in *Escherichia coli,* which is an inexpensive mass production system.

When such human monoclonal antibody gene, chimeric antibody gene or scFv-Fc antibody gene, for example, is used as the gene to be introduced into birds according to the invention, those antibody drugs which have been difficult to produce can be produced in large quantities in the G0 transgenic chimeric birds of the invention.

In the case of G0 transgenic chimeric birds obtained by introduction of a chimeric antibody gene, for example, the antibody content in blood is preferably not lower than 0. 5 μg/ml, more preferably not lower than 5 μg/ml. The antibody

content in egg white is preferably not lower than 0.1 μg/ml, more preferably not lower than 1 μg/ml, and the antibody content in egg yolk is preferably not lower than 0.1 μg/ml, more preferably not lower than 1 μg/ml.

In the case of G0 transgenic chimeric birds resulting from introduction of an scFv-Fc gene, the antibody content in blood is preferably not lower than 20 μg/ml, more preferably not lower than 2000 μg/ml. The antibody content in egg white is preferably not lower than 5 μg/ml, more preferably not lower than 500 μg/ml. The antibody content in egg yolk is preferably not lower than 1 μg/ml, more preferably not lower than 100 μg/ml. While the antibody accumulated in the yolk is decomposed enzymatically, for example, the above-mentioned content is the value given under assumption that the antibody does not undergo such decomposition. The antibody decomposition in egg yolk can be prevented, for example, by adding an enzyme inhibitor at an early stage or by modifying the structure susceptible to decomposition.

As the above-mentioned promoter gene, there may be mentioned constitutive promoters. In cases where the antibody gene is controlled under a constitutive promoter, the antibody gene expression is favorably stable. As a more preferred constitutive promoter, there may be mentioned the chicken β-actin promoter. When the expression is controlled under the β-actin promoter, the desired protein is characteristically expressed in the blood.

When, in the protein production in transgenic animals, the desired protein is produced in milk or in eggs, the protein production level cannot be estimated until the start of lactation or egg laying as a result of sexual maturation; this is a disadvantage. Several years is required for sexual maturation in some cases depending on the animal species and this is an unstable factor in planned commercial production of a desired protein. In this respect, the use of transgenic birds in which the expression of the protein is controlled under the β-actin promoter makes it possible to predictably know the level of protein expression in eggs after sexual maturation by examining the level of protein expression in blood in the stage of juvenile birds. To sort out birds based on the results obtained will serve as a positive factor in planned protein production.

The protein production method is characterized in that a desired protein is recovered from the above-mentioned transgenic bird. More particularly, it is a method of protein production which comprises recovering and purifying the desired protein from somatic cells, blood and/or eggs of the transgenic bird constructed. Further, the eggs laid by the transgenic bird also constitute an aspect of the present disclosure The content of the desired protein in the above-mentioned eggs is preferably not lower than 1 mg/100 g, more preferably not lower than 20 mg/100 g, particularly preferably not lower than 100 mg/100 g.

Now, methods for constructing G0 transgenic chimeric birds which are parents to the G1 transgenic birds and offspring are described.

As the construction method of the invention, the construction method comprises incubating fertilized avian eggs, infecting the early embryo of each egg with the replication-deficient retroviral vector at least 48 hours, after the start of incubation, and allowing the embryo to hatch.

The method comprises microinjecting the replication-deficient retroviral vector into the heart formed in the early embryo mentioned above.

As regards the early development of fertilized eggs after egg laying in chickens, for example, the egg first fertilized in the oviduct begins to cleave in about 1.5 hours after fertilization. After the start of discoidal cleavage while maintaining cytoplasm connection, cleavage continues over a day until the release of the egg from the body and the egg thus forms an embryo called blastoderm consisting of about 60 thousand cells (blastodermic stage). This blastoderm is observed as a white ring with a diameter of 3 to 4 mm in the middle of the yolk. This embryo divides into the upper and lower layers to form a segmentation cavity. Egg laying occurs nearly at the time of hypoblast formation, a primitive streak is formed, the blastoderm takes a three (upper, median and lower) layer structure, and three germ layers are thus formed. Thereafter, embryo formation and growth continues, and the egg hatches out on the 22nd day after oviposition. The blastodermic stage is also called stage X and reproductive cells are formed from part of cells at this stage and, therefore, the fertilized egg in this stage is used in the prior art as the target of gene transfer.

The present inventors placed fertilized eggs in the blastodermic stage just after egg laying under incubation conditions, for example under environmental conditions suited for incubation in the case of chickens, for example, namely at a temperature of 37.7 to 37.8°C and a humidity of about 50 to 70%, and made microinjection at timed intervals, with the time of placing under such incubation conditions being taken as hour 0. After 36 hours after the start of incubation in the case of quails or after about 50 hours after the start of incubation in the case of chickens, the formation of a blood circulatory system was observed on the yolk and the pulsation of the organ to differentiate into the heart could be observed.

Applicable to the incubation of the above-mentioned fertilized egg after gene introduction is, for example, the method using an artificial eggshell as developed by the present inventors (Kamihara, M. et al. (1998), Develop. Growth Differ., 40, 449).

As the replication-deficient retroviral vector, the gene to be introduced and the bird to be rendered transgenic, which are to be used in the construction method of the invention, there may be mentioned the same ones as mentioned above referring to the G0 transgenic chimeric bird.

In the construction method of the invention, the replication-deficient retroviral vector contains a non-retrovirus-derived gene.

In the construction method of the invention, the "non-retrovirus-derived gene" encodes an antibody

The non-retrovirus-derived gene is preferably one under the control of the chicken β-actin promoter.

In the construction method of the invention, it is preferable to microinject a replication-deficient retroviral vector preferably having a titer of not lower than 1 x $10^7$ cfu/ml, more preferably not lower than 1 x $10^8$ cfu/ml, still more preferably not lower than 1 x $10^9$ cfu/ml, because the gene can then be introduced efficiently.

In the construction method of the invention, the bird resulting from a fertilized egg with a gene introduced therein grows as a transgenic bird carrying the transgene mosaically in somatic cells thereof. This first generation transgenic bird is called G0 transgenic chimeric bird.

The second generation or third generation born upon mating of such a G0 transgenic chimeric bird with a non-transgenic bird or mating G0 transgenic chimeric birds with each other, when developed from a reproductive cell having the transgene on a chromosome thereof, grows as an individual whose somatic cells in the whole body contain the transgene. The offspring inheriting the transgene from this reproductive lineage chimeric individual (s) are referred to, from generation to generation, as G1, G2, G3 transgenic birds and the like.

By mating the G0 transgenic chimeric bird constructed in accordance with the invention with a non-transgenic bird of the same species or a mating type G0 transgenic chimeric bird, it is possible to propagate the transgene to offspring thereof and, at the same time, construct perfect transgenic birds carrying the transgene in somatic cells in the whole body. By establishing a transgenic bird line capable of stably propagating the transgene in that way, it becomes possible to stabilize the quality thereof as a protein production system. Furthermore, a perfect transgenic bird, which has a high proportion of transgene-carrying somatic cells, can be expected to give an increased production of the transgene-due recombinant protein in some cases as compared with the G0 transgenic chimeric bird.

The method for constructing G1 transgenic birds according to the invention comprises constructing the G0 transgenic chimeric bird mentioned above and mating a G0 transgenic male and a wild type female. Further, backcross breeding using offspring and parents thereof is also possible.

By collecting sperm from the G0 male and checking the same as to the occurrence or non-occurrence of the transgene therein, it is possible to sort out reproductive lineage chimeras. Conceivable methods of testing for the transgene include, but are not limited to, the PCR technique and Western blot technique, and the like.

A method for sorting out reproductive lineage chimeras which comprises collecting sperm samples from male G1 transgenic birds, male G2 transgenic birds or male offspring thereof and testing them for the gene in the sperm in the same manner is also disclosed.

When the reproductive lineage chimera is a male, the chimera occurs as an individual having both spermatozoa carrying the transgene and spermatozoa not carrying the transgene. A young bird that has matured from an embryo fertilized with a spermatozoon carrying the transgene becomes a G1 transgenic carrying the transgene in all somatic cells thereof. By sorting out reproductive lineage chimeric G0 birds, it becomes possible to construct G1 transgenic chimeric birds more efficiently as compared with random mating.

(EFFECTS OF THE INVENTION)

The G1 transgenic bird and offspring thereof can efficiently express a foreign gene introduced by means of a highly safe, replication-deficient retroviral vector, without inactivation of the expression product.

Further, the G1 transgenic birds carry the transgene as existing in all somatic cells and allow stable expression of the corresponding protein on the individual level and, further, such birds, as an established lineage, can serve as a stable supplying system.

Furthermore, the method for constructing transgenic birds according to the invention makes it possible to safely and efficiently construct G1 transgenic birds and, further, as offspring thereof, G2 and G3 transgenic birds.

The G1, G2, G3 and subsequent generation transgenic birds express the foreign gene-due protein in somatic cells thereof and, when the protein is recovered and purified from the serum, egg white and/or egg yolk, those birds can supply an inexpensive production system for sugar chain-added proteins or antibodies that have so far been difficult to produce.

BRIEF DESCRIPTION OF THE DRAWINGS

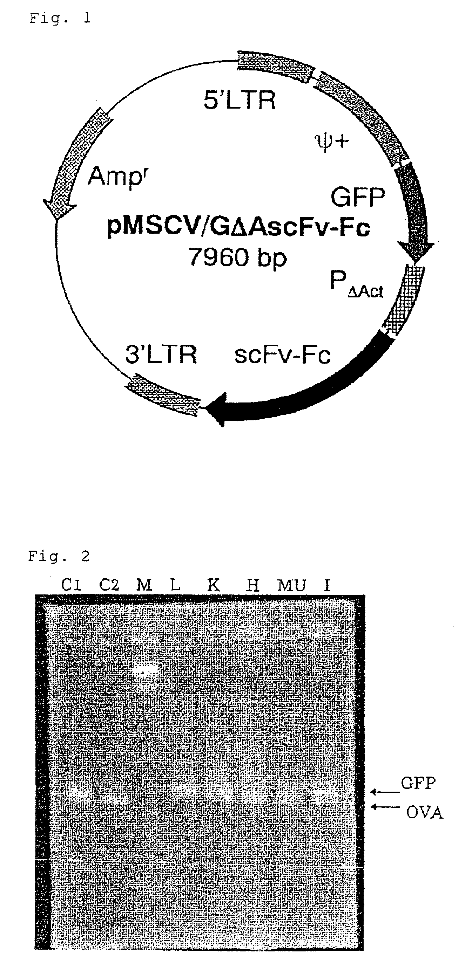

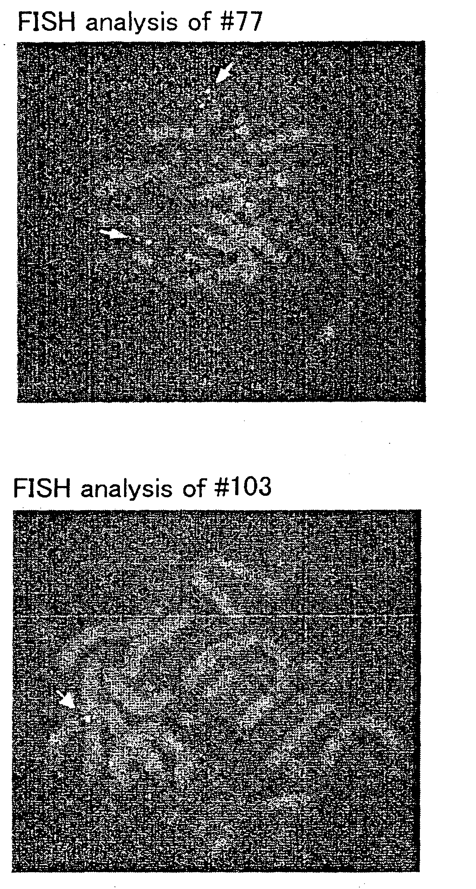

[Fig. 1] This figure shows the structure of the scFv-Fc antibody expression vector construct pMSCV/GΔAscFv-Fc. Amp[r] indicates the ampicillin resistance gene. PΔact indicates the β-actin promoter gene. ψ+ indicates a packaging signal sequence. GFP indicates the green fluorescent protein gene. scFv-Fc indicates the scFv-Fc antibody gene. 5' LTR and 3'LTR respectively indicate the long terminal repeat sequences of MoMLV.

[Fig. 2] This figure shows the results of analysis, by PCR, of the transgene in genome extracts from various organs of a G1 transgenic chicken. M indicates a marker, C1 a positive control, and C2 a negative control. L, K, H, MU and I indicate liver, kidney, heart, muscle and intestine, respectively. GFP indicates the green fluorescent protein gene, and OVA indicates the ovalbumin gene.

[Fig. 3] This figure shows the results of FISH analysis of the G1 transgenic chickens #77 and #103. Each arrow indicates the transgene (pMSCV/scFv-FΔ6BamHI).

BEST MODES FOR CARRYING OUT THE INVENTION

The following examples illustrate the present invention in detail. These examples are, however, by no means of the scope of the present invention.

(Example 1) Formation of a scFv-Fc antibody expression vector construct

A scFv-Fc antibody expression vector construct was prepared in the following manner.

1. A fragment containing a series of the murine phosphoglycerate kinase (PGK) promoter and the Neo$^r$ gene was eliminated from pMSCVneo (product of Clontech Laboratories, Inc.) using the restriction enzymes BglII and BamHI, and the remaining vector fragment was allowed to self-ligate to construct the plasmid pMSCV.

2. A GFP gene fragment was excised from pGREEN LANTERN-1 (product of Gibco BRL) with the restriction enzyme NotI and inserted into pZeoSV2(+) (product of Invitrogen Corporation) at the NotI site. A plasmid having a structure resulting from insertion of the GFP gene in the same direction as the T7 promoter was named pZeo/GFP.

3. A GFP gene fragment was excised from pZeo/GFP with the restriction enzymes EcoRI and XhoI and joined to a vector fragment of pMSCV treated with the restriction enzymes EcoRI and XhoI to construct a plasmid, pMSCV/G.

4. A ΔAct promoter fragment was amplified from pMiwZ (Suemori et al., 1990, Cell Diff. Dev. 29:181-185) by PCR (94°C/15 seconds → 50°C/30 seconds → 68°C/1 minute: 10 cycles; 94°C/15 seconds - 62°C/30 seconds → 68°C/1 minute: 30 cycles; KOD-Plus-DNA polymerase (product of Toyobo Co., Ltd.)) using, as primers, two chemically synthesized oligonucleotides, 5'-acgcgtcgacgtgcatgcacgctcattg-3' (SEQ ID NO:1, the underlined portion being the SalI restriction enzyme site) and 5'-acgcgtcgacaacgcagcgactcccg-3' (SEQ ID NO:2, the underline portion being the SalI restriction enzyme site). The ΔAct promoter fragment was then excised using the restriction enzyme SalI and inserted into pETBlue-2 (product of Novagen) at the SalI site thereof, whereby a plasmid, pETBlue/ΔAct, was constructed.

5. The ΔAct promoter fragment was excised from pETBlue/ΔAct with the restriction enzyme SalI and inserted into pMSCV/G at the XhoI site thereof. A plasmid having a structure resulting from insertion of the ΔAct promoter in the same direction as the GFP gene was named pMSCV/GΔA.

6. A lysozyme secretion signal gene fragment was prepared by annealing together two 5'-terminally phosphorylated chemically synthesized oligonucleotides, 5'-ctagaccatgaggtctttgctaatcttggtgctttgcttcctgcccctggctgctc tgggg-3' (SEQ ID NO:3, the underlined portion being the XbaI terminal and the underlined portion being the HaeIII terminus) and 5'-ccccagagcagccaggggcaggaagcaaagcaccaagattagcaaagacctcatgg t-3' (SEQ ID NO:4, the underlined portion being the XbaI terminal and the underlined portion being the HaeIII terminal) . A scFv gene fragment was amplified from the plasmid pPDS/scFv (Nakamura et al., 2000, Cytotechnology 32:191-198) containing a single chain antibody (scFv) gene prepared from the chicken antibody variable region gene of HUC213 cells by PCR (94°C/15 seconds → 58°C/30 seconds → 68°C/1 minute: 30 cycles) using, as primers, two chemically synthesized oligonucleotides, 5'-gcgtttaaagtgacgttggacgtccg-3' (SEQ ID NO:5, the underlined portion being the DraI restriction enzyme site) and 5'-attaggatccgcgcttaaggacggtcagg-3' (SEQ ID NO:6, the underlined portion being the BamHI restriction enzyme site), and the gene fragment was then excised using the restriction enzymes DraI and BamHI. The two fragments prepared in the above manner were inserted into pBluescriptIISK(+) (product of Stratagene) into the XbaI-BamHI site thereof to construct a plasmid, pBlue/scFv.

7. A scFv gene fragment was excised from pBlue/scFv with the restriction enzymes NotI and BamHI and inserted into pCEP4 (product of Invitrogen Corporation) at the NotI-BamHI site thereof to construct a plasmid, pCEP4/scFv.

8. mRNA was collected from the human IgG1-producing myeloma cells IM-9 (Japanese Collection of Research Bioresources 0024) using a mRNA isolation kit (product of Roche), and a cDNA library was constructed from the mRNA obtained using ReverTra Ace (product of Toyobo Co., Ltd.). A hCγ1 gene fragment was amplified from the above cDNA library by PCR (95°C/2 minutes → 52°C/30 seconds - 74°C/3 minutes: 30 cycles; Pfu DNA polymerase (product of Promega)) using, as primers, two chemically synthesized oligonucleotides, 5'-caagcttcaagggccat-3' (SEQ ID NO:7) and 5'-atttacccggagacaggga-3' (SEQ ID NO:8). Further, a human antibody H chain γ1 Fc region (Fc) gene fragment was amplified from the above PCR product by PCR.(94°C/15 seconds → 58°C/30 seconds →68°C/1 minute: 30 cycles; KOD-plus-DNA polymerase) using, as primers, two chemically synthesized oligonucleotides, 5'-attaggatccgagcccaaatcttgtgacaaaactc-3' (SEQ ID NO:9, the underlined portion being the BamHI restriction enzyme site) and 5'-agc<u>aagctt</u>tcatttacccggagacaggga-3' (SEQ ID NO:10, the underlined portion being the HindIII restriction enzyme site), followed by excision with the restriction enzymes BamHI and HindIII. The excised fragment was inserted into pBluescriptIISK(+) at the BamHI-HindIII site to construct a plasmid, pBlue/Fc.

9. A scFv gene fragment was excised from pCEP4/scFv with the restriction enzymes HindIII and BamHI. A Fc gene fragment was excised from pBlue/Fc with the restriction enzymes BamHI and HindIII. The two fragments thus excised were inserted into pBluescriptIISK (+) at the HindIII. site to construct a plasmid, pBlue/scFv-Fc.

10. A gene fragment having a structure resulting from connection of the human antibody H chain $\gamma 1 \cdot$Fc to the chicken single chain antibody variable region was excised from pBlue/scFv-Fc with the restriction enzyme HindIII and ligated to a vector fragment prepared by treatment of pMSCV/G$\Delta$A with the restriction enzyme HindIII. A plasmid having a structure resulting from joining of the scFv-Fc gene in the same direction as the $\Delta$Act promoter was named pMSCV/ G$\Delta$AscFv-Fc.

The structure of the thus-constructed replication-deficient retroviral vector-derived vector construct pMSCV/ G$\Delta$AscFv-Fc is shown in Fig. 1.

(Example 2) Preparation of a scFv-Fc antibody expression retroviral vector

For preparing a retroviral vector from the vector construct pMSCV/G$\Delta$AscFv-Fc constructed in Example 1, 5 x $10^6$ packaging cells GP293 (product of Clontech Laboratories, Inc.) were sown and cultured in a culture dish having a diameter of 100 mm. The medium was replaced with fresh DMEM (Dulbecco's modified Eagle medium), and 8 $\mu$g of the pVSV-G vector (product of Clontech Laboratories, Inc.) and 8 $\mu$g of pMSCV/G$\Delta$AscFv-Fc were introduced into the above GP293 cells by the lipofection technique. After 48 hours, the culture supernatant containing viral particles was recovered and deprived of contaminants by passing through a 0.45-$\mu$m cellulose acetate filter (product of Advantech Co., Ltd.). Polybrene (product of Sigma) was added to the solution obtained to a concentration of 10 $\mu$g/ml and the resulting mixture was used as a virus solution.

The virus solution prepared was added to separately cultured GP293 cells and, after 48 hours of cultivation, the cells were cloned by the limiting dilution method. A stable transformant GP293 strain capable of strongly expressing GFR was thus obtained.

The stable transformant strain obtained was cultured in a dish with a diameter of 100 mm until an 80% confluent state, and 16 $\mu$g of pVSV-G was introduced thereinto by the lipofection technique. After 48 hours, 12 ml of a culture supernatant containing viral particles was recovered.

This culture supernatant was centrifuged at 50,000 x-g and at 4°C for 1.5 hours to settle the viral particles. The supernatant was removed, 50 $\mu$l of a solution containing 50 mM Tris-HC1 (pH 7.8) 130 mM NaCI and 1 mM EDTA was added to the viral particle-containing precipitate and, after overnight standing at 4°C and after thorough suspending, a virus solution was recovered. The thus-obtained high-titer viral vector showed a titer of $10^8$ to $10^9$ cfu/ml.

The viral titer was measured in the following manner. On the day before measurement, 7 x $10^4$ NIH3T3 cells (obtained from the American Type Culture Collection) were sown and cultured in each dish with a diameter of 35 mm. A $10^2$- to $10^6$-fold diluted virus solution (1 ml) was added to each dish and, after 48 hours, the proportion (100% being taken as 1) of cells expressing GFP was determined using a fluorescent microscope. The titer was determined according to the following calculation formula:

```
Viral titer = number of cells x dilution factor x proportion
of expression (cfu/ml)
```

(Example 3) Injection of the retroviral vector into chicken embryos

Fertilized chicken eggs (Nippon Institute for Biological Science) were used. These fertilized eggs were placed in a 37.9°C and 65% humidity environment in an incubator (Showa Furanki model P-008) with a built-in automatic egg-turning device. The time of such placement was taken as the incubation start time (zero hour) and, thereafter, incubation was performed while rotating the eggs 90 degrees at 15-minute intervals.

For gene injection, the eggshell of each fertilized egg was disinfected with 70% ethanol and a circle portion with a diameter of 3.5 cm was cut off from the sharp end portion thereof with a diamond cutter (Minomo 7C710, product of MINITOR CO.; LTD) to expose the embryo. At about the 50th hour after the start of incubation, blood vessel development was observed on the egg yolk surface and it could be observed under a stereoscopic microscope that a part thereof pulsated, thus becoming a primordium of the heart. While observing such initial stage heart under a stereoscopic microscope, a needle manufactured by processing a glass tube (CD-1, product of OLYMPUS CORPORATION) using a micropipette manufacturing apparatus (PC-10, product of OLYMPUS CORPORATION) and breaking the tip so that the outside diameter might become about 20 $\mu$m was stuck into the middle of the cavity beneath the germinal disk and about 2 $\mu$l of the virus solution prepared in Example 2 was microinjected into that cavity using a microinjector (Transjector 5246, product of Eppendorf Co., Ltd.). The eggshell was filled with egg white to the opening made by cutting, and the opening was covered with a Teflon membrane (MilliWrap, product of Millipore Corporation) and a polyvinylidene chloride wrap (Saran Wrap, product of Asahi Kasei Corporation) using egg white as a paste. Incubation was then performed while rotating the egg 90 degrees at 30-minute intervals.

On the 18th day after the start of incubation, the egg turning was stopped and, on the 21st day, when the chick began pecking, the eggshell was artificially broken to assist hatching. The hatchability in such artificial hatching was 50 to 60%.

(Example 4) Serum scFv-Fc antibody assaying by ELISA

The G0 transgenic chimeric chicks born in Example 3 were fed to grow. After one week, blood samples were collected from the grown male chicks (individual identification numbers: # 1111, #3108 and #4102) via the vein under the wing. Each blood sample obtained was centrifuged at 15,000 rpm for 10 minutes, and the serum obtained as the supernatant was assayed for scFv-Fc antibody titer.

An anti-human IgG antibody (product of Cosmo Bio Co., Ltd.) diluted with PBS was distributed in 100-$\mu$g portions into wells of an ELISA plate, followed by overnight standing at 4°C. Each well was washed with three 200-$\mu$l portions of a 0.05% Tween 20 solution in PBS and then 150 $\mu$l of Pubs-0.05% Tween 20 to 2% skimmed milk was added to each well.

After 2 hours of standing at room temperature, each well was washed with three 200-$\mu$l portions of PBS-0.05% Tween 20, and 120 $\mu$l of the blood sample collected was added thereto, followed by overnight standing at 4°C. This ELISA plate was returned to room temperature, each well was washed with three portions of the PBS-Tween 20 solution, 100 $\mu$l of peroxide (POD)-labeled anti-human IgG antibody (product of Cosmo Bio Co., Ltd.) diluted with PBS-0.05% Tween 20 was added to each well, and the plate was allowed to stand at room temperature for 1 hour.

Each well was washed with four portions of Pubs-0 . 05% Tween 20, and 100 $\mu$l of a color developer solution (prepared by dissolving 10 mg of o-phenylenediamine (product of Katayama Chemical Industries Co., Ltd.) in 1 ml of methanol, making the volume to 100 ml with distilled water and adding 10 $\mu$l of an aqueous hydrogen peroxide solution (product of Wako Pure Chemical Industries Ltd.) ) was added to each well. The reaction was terminated by adding 50 $\mu$l of 8 M sulfuric acid to each well, the fluorescence intensity at 490 nm was measured using a plate reader, and the concentration was determined based on a standard working curve. The mean of the antibody concentrations in the samples obtained from three quails or chicks was reported as the result.

The standard antibody (product of Cosmo Bio Co., Ltd.) for standard working curve construction was diluted with 50% egg yolk-PBS (w/v).

The standard working curve was constructed using purified scFv-Fc. The vector construct pMSCV/scFv-Fc constructed in Example 1 was introduced into GP293 cells by the lipofection technique, and the culture supernatant derived therefrom was centrifuged (4°C, 10 minutes, 3,000 rpm) to remove the solid matter. While cooling and stirring this supernatant, ammonium sulfate finely ground to 50% saturation was gradually added (313 g ammonium sulfate/1, 000 ml of water) for protein precipitation. This was allowed to stand overnight at 4°C and then centrifuged at 15, 000 rpm and at 4°C for 10 minutes to cause the precipitate to wholly settle. The precipitate was dissolved in a small amount of PBS. The solution was dialyzed against 2 L of PBS three times to remove ammonium sulfate.

A protein G column for purification (product of NGK INSULATORS LTD.) was initially washed with 10 mL of Binding Buffer (NaHPO$_4$•2H$_2$O 1.56 g/l, NaHPO$_4$•12H$_2$O 7.16 g/l), 10 ml of Wash Buffer (acetic acid 20%, distilled water 80%) and 10 ml of Binding Buffer in that order (flow rate 2 ml/minute). A solution of the protein dissolved in PBS was passed through the column at 1 ml/minute for scFv-Fc to be adsorbed on the column. Unnecessary proteins were removed by passing 20 ml of Binding Buffer at 1.7 ml/minute, and the scFv-Fc was eluted by passing Elution Buffer (glycine 7.507 g/adjusted to pH 2.5 to 3.0 with 1.2 N HCl) at 1.5 ml/minute.

Each eluate fraction was dialyzed against PBS (2 L) three times and the thus-purified scFv-Fc was assayed for protein concentration based on the absorbance at the wavelength 280 nm. The blood antibody levels in the three male G0 transgenic chimeric chicks born in Example 3 (individual identification numbers. #1111, #3108 and #4102) are shown in Table 1.

Table 1

| Individual Id. No. | Blood antibody level |
|---|---|
| #1111 | 1.6mg/mL |

(continued)

| Individual Id. No. | Blood antibody level |
|---|---|
| #3108 | 1.6mg/mL |
| #4102 | 1.5mg/mL |

(Example 5) Confirmation of the transgene in sperm by PCR

The three male G0 transgenic chimeric chicks born in Example 3 (individual identification numbers #1111, #3108 and #4102) were fed and grown, waiting for sexual maturation. After 6 months, 0.2 mL of sperm was collected from each of the grown male transgenic chimeric chickens by abdominal massage.
The transgene was confirmed by the PCR method using 50 ng of the genomic DNA extracted from each collected sperm sample using a kit (product of Toyobo Co., Ltd., MagExtractor-genome-).
A set of primers enabling amplification of a part (393 bp) of the scFv gene fragment contained in the transgene: 5'-GTCTTATTAGCGGTGCTGGTAGTAGCACAA-3' (SEQ ID
NO:11)/5'-GAGACTTCTGCTGGTACCAGCCATA-3' (SEQ ID NO:12) and a set of primers enabling amplification of a part (311 bp) of the GFP gene contained in the transgene: 5'-AGCTCACCCTGAAATTCATCTGCACCACTG-3' (SEQ ID NO:13)/5'-GTTGTATTCCAGCTTGTGGCCGAGAATGTT-3' (SEQ ID NO:14) were used in the PCR.

(Example 6) Construction of G1 transgenic chickens by mating

A male G0 transgenic chimeric chicken (#4102) in which the transgene had been detected in the sperm in Example 5 was allowed to mate with three wild type females (white Leghorn), and fertilized eggs were obtained.
These fertilized eggs were incubated in a 37.9°C and 65% humidity environment in an incubator (Showa Furanki model P-008) with a built-in automatic egg-turning device and, on the 21st or 22nd day, chicks were born. These chicks were fed for a week and 100-μl blood samples were collected from the vein under the wing. The serum was separated from each blood sample and the antibody level in the serum was determined by the ELISA method described in Example 4. Further, the genome was extracted from each blood sediment obtained by centrifugation by the method described in Example 5, and the transgene was confirmed by the PCR method.
A set of primers enabling amplification of a part (355 bp) of the GFP gene contained in the transgene: 5'-CAACACTGGTCACTACCTTCACCTATG-3' (SEQ ID N0:15)/5'-ACGGATCCATCCTCAATGTTGTGTC-3' (SEQ ID NO:16) were used in the PCR. As an internal standard, a set of primers enabling amplification of a part (317 bp) of the ovalbumin gene contained in the chicken genome: 5'-CGCTTTGATAAACTTCCAGGATTCGG-3' (SEQ ID NO:17)/5'-CATCTAGCTGTCTTGCTTAAGCGTACA-3' (SEQ ID NO:18) were used.
As a result of transgene testing in 110 chicks, the transgene was detected in 5 chicks. The genome was extracted from each organ of a G1 individual that had accidentally died, and the genome was subjected to transgene confirmation testing by PCR.

(Example 7) Confirmation of the transgene in each organ in G1

Since one of the G1 individuals constructed in Example 6 and confirmed to have the transgene in the genome extracted from the blood accidentally died, the stomach, liver, kidney, intestine and muscle were excised, and the genome was extracted from each organ by the method described in Example 6 and subjected to transgene confirmation testing by the PCR method (Fig. 2).
The results obtained positively proved that this individual was a G1 individual carrying the transgene in each organ in the body.
The blood scFv-Fc antibody levels in the blood samples from the five G1 chicks were determined by the method described in Example 4. In three chicks among them, the antibody expression level was 1 mg/mL.
It was thus revealed that this method makes it possible to construct perfect G1 transgenic chickens capable of expressing a transgene-due scFv-Fc antibody and carrying the transgene in all somatic cells.

(Example 8) G1 chromosome analysis by FISH

The 77th chick that had hatched in Example 6 was found to be a G1 transgenic chick (female) by the PCR testing described in Example 6. This G1 is given the individual number #77. In the same manner, the 103rd chick was found to be a G1 transgenic chick (male) by the PCR method (individual number #103).
Blood samples were collected from these two G1 chicks via the vein under the wing, and lymphocytes were separated from 2 mL of each blood sample by the Ficoll-Hypaque method and cultured in a 5% $CO_2$ atmosphere at 37°C using RPMI 1640 supplemented with a mitogen (concanavalin A, product of Cosmo Bio Co., Ltd.), mercaptoethanol (product of Wako Pure Chemical Industries Ltd.), kanamycin sulfate (product of Cosmo Bio Co., Ltd.) and 18% FBS (product of Invitrogen Corporation). Several hours prior to chromosome specimen preparation, BrdU (5-bromodeoxyuridine, product of Merck Ltd.) was added to the medium, colcemid (product of Merck Ltd.) was further added and, after 1 hour, cells were recovered, treated with a hypotonic solution and fixed with a fixative solution (methanol:acetic acid = 3:1) to give a specimen.

Each chromosome specimen prepared was dried for several days, stained with Hoechst 33258 (product of Merck Ltd.) and, after UV treatment and drying, analyzed by FISH. A probe DNA (pMSCV/scFv-FcΔBamHI) was labeled with biotin (product of Cosmo Bio Co., Ltd.), and a goat anti-biotin antibody/FITC-anti-goat IgG system was used for the detection reaction, imaging was effected using a fluorescence microscope (Leica DMRA system), and analysis was performed using an image analyzer (Leica 550CW-QFISH software).

The probe DNA (pMSCV/scFv-FcABamHI) was prepared by excising a BamHI-BamHI fragment (1.57 kb) from pMSCV/scFv-Fc of Example 1 by restriction enzyme treatment and labeling with biotin in the conventional manner.

The thus-obtained results of FISH analysis of #77 and #103 are shown in Fig. 3. It was revealed from these results of FISH analysis that the #77 individual was a G1 carrying two copies of the transgene as inserted in the microchromosome and that #103 was a G1 transgenic chick carrying one copy of the transgene as inserted in the long arm of the third chromosome.

(Example 9) Construction of G2 transgenic chickens by mating

The G1 #77 individual (female) of Example 8 was mated with a wild type male (white Leghorn) and the #103 individual (male) with a wild type female (white Leghorn), and the fertilized eggs obtained were allowed to hatch. The genome was extracted from plasma of each chick by the method of Example 5 and analyzed as to the presence or absence of the transgene by the PCR method; thus, each chick was tested as to whether it was a G2 transgenic chick.

Since #77 was a G1 carrying 2 copies of the transgene in one of the chromosomes, it was expected from the probability viewpoint that three fourths of chicks born as a result of mating with a wild type be transgenic. One fourth ought to be G2 chicks carrying two copies of the transgene, like the G1 parent, and two fourths to be G2 chicks carrying only one of the transgene copies which the G1 parent had.

Upon actual examination of the chicks that had hatched, it was found that 14 out of 20 chicks were G2 transgenic ones, namely that G2 chicks had been born roughly with a frequency expected from the probability viewpoint.

In the same way, the chicks born from mating of G1 #103 with a wild type were tested by the PCR method. Since #103 was a G1 carrying one copy of the transgene, it was anticipated that G2 chicks should appear with a probability of 1/2. The results of actual testing showed that 22 out of 43 chicks were G2 transgenic. Thus, it was found that G2 chicks as expected from the probability principle can be born from transgenic G1 chickens constructed in accordance with the present invention.

The G2 chicks constructed were raised, and the antibody levels in eggs laid by females and the expression levels in blood in the females were measured by the method of Example 4. The blood antibody expression levels in G2 individuals (6 months of age), which were offspring of G1 #77 and #103, are shown in Table 2 and Table 3, respectively, and the antibody expression levels in eggs laid by 6-month-old G2 females born from #77 are shown in Table 4.

Table 2

| Blood scFv-Fc antibody levels in G2 from #77 | |
|---|---|
| Individual Id. No. | Blood antibody level |
| #77-3 | 0. 63mg/mL |
| #77-5 | 0.58mg/mL |
| #77-22 | 0.75mg/mL |

Table 3

| Blood scFv-Fc antibody levels in G2 from #103 | |
|---|---|
| Individual Id. No. | Blood antibody level |
| #103-3 | 0.35mg/mL |
| # 103-8 | 0.42mg/mL |

(continued)

| Blood scFv-Fc antibody levels in G2 from #103 | |
|---|---|
| Individual Id. No. | Blood antibody level |
| #103-16 | 0.38mg/mL |

Table 4

| scFv-Fc antibody levels in eggs laid by G2 from #77 | | |
|---|---|---|
| Individual Id. No. | Antibody level in egg white | Antibody level in egg yolk |
| #77-12 | 0.57mg/mL | 0. 33mg/mL |

While G1 #77 showed a blood antibody level of 0.5 to 0.8 mg/mL, the G2 chicks born as a result of mating thereof with a wild type showed nearly the same blood antibody levels, as shown in Table 2. While G1 #103 showed a blood antibody level of 0.3 to 0.5 mg/mL, the G2 chicks born as a result of mating thereof with a wild type showed almost the same blood-antibody levels, as shown in Table 3. Further, in the case of the G2 female from #77 which gave an average antibody expression level in egg white of 0.3 mg/mL, the antibody was expressed in the egg white in eggs therefrom at almost the same levels, as shown in Table 4.

These results demonstrated that the transgenic chicken of the invention will not undergo gene silencing even through a number of generations and, when an increased number of offspring produced from that transgenic by mating are used, they can be applied as a low-cost protein production factory.

INDUSTRIAL APPLICABILITY

The G1 transgenic bird and offspring thereof can express efficiently and without inactivation a foreign gene introduced by means of a highly safe, replication-deficient retroviral vector.

The G1 transgenic bird carries the transgene in all somatic cells, allows stable protein expression on the individual level and, as an established line, can become a stably protein source.

Further, the method for constructing transgenic birds according to the invention makes it possible to safely and efficiently construct G1 transgenic birds and, further, G2 and G3 transgenic birds as offspring thereof.

The G1, G2, G3 and subsequent generation transgenic birds express a foreign gene-due protein in somatic cells and, when the protein is recovered and purified from serum, egg white and/or egg yolk, an inexpensive production system for sugar chain-carrying proteins, which have so far been difficult to produced, or antibodies can be provided.

## Claims

1. A method for constructing a G1 transgenic bird which comprises:

   a) incubating a fertilized avian egg,
   b) microinjecting into the heart of the early embryo thereof after the blastodermic stage when the early embryo is at least 48 hours after the start of incubation, a replication-deficient retroviral vector containing a non-retrovirus-derived gene coding for an antibody,
   c) allowing the egg to hatch out to thereby obtain a G0 transgenic chimeric bird, and
   d) mating a Male G0 transgenic chimeric bird
   with a wild-type Female bird.

2. The method for constructing a transgenic bird according to claim 1, wherein the bird is a chicken or a quail.

3. The method for constructing a transgenic bird according to any one of Claims 1 and 2 which comprises microinjecting a replication-deficient retroviral vector having a titer of not lower than 1 x $10^7$ cfu/ml.

4. The method for constructing a transgenic bird according to Claim 3 which comprises microinjecting a replication-deficient retroviral vector having a titer of not lower than 1 x $10^9$ cfu/ml.

5. The method for constructing a transgenic bird according to any one of Claims 1 to 4, wherein the replication-deficient retroviral vector is a vector derived from Moloney murine leukemia virus.

6. The method for constructing a transgenic bird according to any one of Claims 1 to 4, wherein the replication-deficient retroviral vector is VSV-G pseudotyped.

7. The method for constructing a transgenic bird according to any one of Claims 1 to 6, wherein the non-retrovirus-derived gene is controlled under the chicken β-actin promoter.

8. The method for constructing a transgenic bird according to any one of Claims 1 to 6, wherein the antibody is a chimeric antibody.

9. The method for constructing a transgenic bird according to Claim 8, wherein the chimeric antibody is scFv-Fc antibody.


**Patentansprüche**

1. Verfahren zum Hervorbringen eines G1-transgenen Vogels, das umfaßt:

   a) Inkubieren eines befruchteten Vogeleis,
   b) Mikroinjizieren in das Herz des jungen Embryos darin eines replikationsdefizienten retroviralen Vektors, der ein nicht von einem Retrovirus stammendes Gen enthält, das einen Antikörper kodiert, nach dem Blastodermstadium, wenn der junge Embryo mindestens 48 Stunden nach Beginn der Inkubation ist,
   c) Schlüpfenlassen des Eis, um damit einen G0-transgenen chimären Vogel zu erhalten, und
   d) Paaren eines männlichen G0-transgenen chimären Vogels mit einem weiblichen Wildtypvogel.

2. Verfahren zum Hervorbringen eines transgenen Vogels nach Anspruch 1, wobei es sich bei dem Vogel um ein Huhn oder eine Wachtel handelt.

3. Verfahren zum Hervorbringen eines transgenen Vogels nach einem der Ansprüche 1 und 2, welches das Mikroinjizieren eines replikationsdefizienten retroviralen Vektors umfaßt, der einen Titer von mindestens 1 x $10^7$KBE/ml aufweist.

4. Verfahren zum Hervorbringen eines transgenen Vogels nach Anspruch 3, welches das Mikroinjizieren eines replikationsdefizienten retroviralen Vektors umfaßt, der einen Titer von mindestens 1 x $10^9$ KBE/ml aufweist.

5. Verfahren zum Hervorbringen eines transgenen Vogels nach einem der Ansprüche 1 bis 4, wobei es sich bei dem replikationsdefizienten retroviralen Vektor um einen Vektor handelt, der vom Moloney-Maus-Leukämievirus abstammt.

6. Verfahren zum Hervorbringen eines transgenen Vogels nach einem der Ansprüche 1 bis 4, wobei der replikationsdefiziente retrovirale Vektor VSV-G-pseudotypisiert ist.

7. Verfahren zum Hervorbringen eines transgenen Vogels nach einem der Ansprüche 1 bis 6, wobei das nicht von einem Retrovirus stammende Gen unter der Kontrolle des β-Aktinpromotors des Huhns steht.

8. Verfahren zum Hervorbringen eines transgenen Vogels nach einem der Ansprüche 1 bis 6, wobei es sich bei dem Antikörper um einen chimären Antikörper handelt.

9. Verfahren zum Hervorbringen eines transgenen Vogels nach Anspruch 8, wobei es sich bei dem chimären Antikörper um einen scFv-Fc-Antikörper handelt.


**Revendications**

1. Procédé pour construire un oiseau transgénique G1 qui comprend :

   (a) l'incubation d'un oeuf aviaire fécondé,
   (b) la micro-injection dans le coeur de l'embryon précoce de celui-ci après le stade blastodermique quand l'embryon précoce est au moins 48 h après le début de l'incubation, d'un vecteur rétroviral défectif pour la réplication contenant un gène non dérivé de rétrovirus codant un anticorps,
   (c) le fait de permettre à l'oeuf d'éclore pour obtenir ainsi un oiseau chimérique transgénique G0, et
   (d) l'accouplement d'un oiseau chimérique transgénique G0 mâle avec un oiseau femelle de type sauvage.

2. Procédé pour construire un oiseau transgénique selon la revendication 1 où l'oiseau est un poulet ou une caille.

3. Procédé pour construire un oiseau transgénique selon l'une quelconque des revendications 1 et 2 qui comprend la micro-injection d'un vecteur rétroviral défectif pour la réplication ayant un titre non inférieur à 1 x $10^7$ cfu/ml.

4. Procédé pour construire un oiseau transgénique selon la revendications 3 qui comprend la micro-injection d'un vecteur rétroviral défectif pour la réplication ayant un titre non inférieur à 1 x $10^9$ cfu/ml.

5. Procédé pour construire un oiseau transgénique selon l'une quelconque des revendications 1 à 4 où le vecteur rétroviral défectif pour la réplication est un vecteur dérivé du virus de la leucémie murine de Moloney.

6. Procédé pour construire un oiseau transgénique selon l'une quelconque des revendications 1 à 4 où le vecteur rétroviral défectif pour la réplication est pseudotypé VSV-G.

7. Procédé pour construire un oiseau transgénique selon l'une quelconque des revendications 1 à 6 où le gène non dérivé de rétrovirus est sous le contrôle du promoteur de β-actine du poulet.

8. Procédé pour construire un oiseau transgénique selon l'une quelconque des revendications 1 à 6 où l'anticorps est un anticorps chimérique.

9. Procédé pour construire un oiseau transgénique selon la revendication 8 où l'anticorps chimérique est un anticorps scFv-Fc.

Fig. 1

Fig. 2

FISH analysis of #77

FISH analysis of #103

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2002176880 A

- JP 2004003045 A

**Non-patent literature cited in the description**

- *Nature Biotechnology,* 2001, vol. 19, 184
- **Harvey A. J. et al.** Consistent production of transgenic chickens using replication-deficient retroviral vectors and high-throughput screening procedures. *Poult. Sci.,* February 2002, vol. 81 (2), 202-212
- **Bosselman, R. A. et al.** *Science,* 1989, vol. 243, 533

- **Powers, D. B. et al.** *J. Immunol. Methods,* 2000, vol. 251, 123
- **Kamihara, M. et al.** *Develop. Growth Differ.,* 1998, vol. 40, 449
- **Suemori et al.** *Cell Diff. Dev.,* 1990, vol. 29, 181-185
- **Nakamura et al.** *Cytotechnology,* 2000, vol. 32, 191-198